# EUROPÄISCHE PATENTSCHRIFT

(11) **EP 0 609 253 B1**
(45) Veröffentlichungstag und Bekanntmachung des Hinweises auf die Patenterteilung: **20.12.1995**
(21) Anmeldenummer: 92920403.0
(22) Anmeldetag: 24.09.1992
(51) Int. Cl.: C07C 33/02, C07C 43/313, C07C 43/315, C07C 29/62, C07C 41/54, C07C 41/48, C07C 29/60

(54) **VERFAHREN ZUR HERSTELLUNG, ZWISCHENPRODUKTE ZUR HERSTELLUNG UND DIE VERWENDUNG EINES GEMISCHES AUS DEN ISOMEREN DES DODECADIENOLS**
PROCESS FOR PREPARING A MIXTURE OF DODECADIENOL ISOMERS, INTERMEDIATES FOR PREPARING THE SAME AND ITS USE
PROCEDE DE FABRICATION D'UN MELANGE COMPOSE D'ISOMERES DE DODECADIENOL, PRODUITS INTERMEDIAIRES POUR CETTE FABRICATION ET APPLICATION DE CE MELANGE

(30) Priorität: 24.10.1991 DE 4135064
(43) Veröffentlichungstag der Anmeldung: 10.08.1994
(73) Patentinhaber: BASF Aktiengesellschaft, 67063 Ludwigshafen (DE)
(72) Erfinder: KLEIN, Ulrich, D-6703 Limburgerhof (DE); NEUMANN, Ulrich, D-6707 Schifferstadt (DE); MACKENROTH, Wolfgang, D-67098 Bad Duerkheim (DE); RENZ, Guenter, D-6800 Mannheim 1 (DE); KRIEG, Wolfgang, D-6721 Weingarten (DE); MACKENROTH, Christiane, D-67098 Bad Duerkheim (DE); BUSCHMANN, Ernst, D-6700 Ludwigshafen (DE); DE KRAMER, Jacobus, Jan, D-76275 Ettlingen (DE); MILLI, Roland, D-6804 Ilvesheim (DE)
(86) Internationale Anmeldenummer: EP9202213
(87) Internationale Veröffentlichungsnummer: WO9308148

(56) Entgegenhaltungen:
- EP-A- 0 003 708
- GB-A- 1 299 691
- SYNTHESIS, Nr. 5, Mai 1981, S. 359-361, Stuttgart, DE; R. ROSSI: "Highly stereoselective synthesis of (Z)-9,11-dodecadien-1-yl acetate; A sex pheronome component of diparopsis castanea HMPS"
- TETRAHEDRON LETTERS, Nr. 18, April 1975, S. 1465-1468, Oxford, GB; J.F.NORMANT: "Synthèse d'énynes et de diènes conjugués à l'aid d'oraganocuivreux vinyliques. Application à la synthèse du bombykol"
- CHEMICAL ABSTRACTS SERVICE, Registry Handbook, Number Section, 1983, Supplement 1983, THE AMERICAN CHEMICAL SOCIETY, Columbus, Ohio, US; RN=85576-23-4
- CHEMICAL ABSTRACTS SERVICE, Registry Handbook, Number Section, 1988, Supplement, 1988, THE AMERICAN CHEMICAL SOCIETY, Columbus, Ohio, US RN=11752-77-9

## Beschreibung

Die Dokumente EP-A-0 003 708 und GB-A-1299691 offenbaren die Herstellung von 8E,10E-Dodecadienol.

Die vorliegende Erfindung betrifft ein Verfahren zur Herstellung eines Gemisches von 8E,10E-Dodecadienol (Ia), 8E,10Z-Dodecadienol (Ib), 8Z,10E-Dodecadienol (Ic) und 8Z,10Z-Dodecadienol (Id),

H₃C-CH=CH-CH=CH-(CH₂)₆-CH₂-OH

(8E,10E = Ia)
(8E,10Z = Ib)
(8Z,10E = Ic)
(8Z,10Z = Id)
welches dadurch gekennzeichnet ist, daß man 1,8-Octandiol (II)

HO-CH₂-(CH₂)₆-CH₂-OH (II)

in an sich bekannter Weise in einem Lösungsmittel in Gegenwart eines Halogenierungsmittels in ein Halogenderivat der allgemeinen Formel (III),

Hal-CH₂-(CH₂)₆-CH₂-OH (III)

in der Hal für ein Halogenatom steht, überführt, (III) anschließend mit einem Vinylether der allgemeinen Formel (IV),

H₂C=CH-O-R (IV)

in der R für einen C-organischen Rest steht, in an sich bekannter Weise in Gegenwart einer Säure zu einem Acetal der allgemeinen Formel (V)

Hal-CH₂-(CH₂)₆-CH₂-O-CH(CH₃)-OR (V)

umsetzt, (V) in Gegenwart von Magnesium mit Crotonaldehyd in ein Alkenol der allgemeinen Formel (VI)

H₃C-CH=CH-CH(OH)-(CH₂)₈-O-CH(CH₃)-OR (VI)

überführt und aus (VI) in Gegenwart von Säure gleichzeitig die Alhoholschutzgruppe abspaltet und die Hydroxyfunktion eliminiert.

Die vorliegende Erfindung betrifft weiterhin neue Zwischenprodukte für dieses Verfahren sowie Verfahren zur Bekämpfung des Apfelwicklers Cydia pomonella nach der Paarungsstörungsmethode mit diesem Gemisch.

Der Apfelwickler ist ein bedeutender Schädling im Apfelanbau.

Bisher wurde der Apfelwickler mit den üblichen Methoden, d.h. durch Anwendung von Insektiziden unspezifisch bekämpft. Eine gezielte Methode konnte nach Identifizierung des Sexuallockstoffes des Apfelwicklers (Roeloffs et al. DE-A 21 23 434) zur Anwendung kommen. Es ist bekannt, daß bei Schmetterlingen von paarungsbereiten weiblichen Tieren Sexuallockstoffe (Pheromone) erzeugt und in die Umgebung ausgeschieden werden. Männliche Schmetterlinge derselben Art können dann mit Hilfe dieses Riechstoffes die Weibchen auffinden.

Grundsätzlich gibt es drei verschiedene Möglichkeiten, Sexuallockstoffe im Pflanzenschutz anzuwenden:

### 1. Monitortechnik

Sogenannte Pheromonfallen, bestückt mit synthetischen Sexuallockstoffködern, werden in potentiellen Befallsgebieten ausgehängt. Der Fallenfang von männlichen Faltern erbringt den Nachweis des Auftretens des Schädlings. Außerdem lassen sich Hinweise zur Befallsstärke und auf den richtigen Zeitpunkt der Bekämpfung ableiten.

### 2. Abfangtechnik

Man kann den Lockstoff mit insektiziden Wirkstoffen kombinieren. Es besteht die Möglichkeit, dem Köder bzw. der Falle Insektizide zuzusetzen oder aber nur in unmittelbarer Umgebung der Falle zu behandeln, damit dann der größte Teil der aus weiter Entfernung angelockten männlichen Falterbevölkerung abgetötet werden kann. Die Biotopbelastung ist auf ein vertretbares Maß reduziert.

### 3. Paarungsstörungsmethode

Schließlich kann der Schädling durch das Verfahren der Luftraumsättigung mit Sexuallockstoffen oder ähnlich wirkenden Substanzen bekämpft werden. Die männlichen Schmetterlinge werden am Auffinden der Weibchen gestört. Dadurch wird die Paarung der Tiere verhindert.

In diesem Fall wird im gesamten Bereich der zu schützenden Pflanzenkultur eine größere Menge des Lockstoffes im Luftraum verteilt, so daß die Männchen überall die Gegenwart des Duftstoffes empfinden können und damit ihr normales Orientierungsverhalten gestört ist.

Vor allen bei der dritten Methode (Paarungsstörungsmethode) handelt es sich um eine äußerst selektive und auch effektive Möglichkeit zur Bekämpfung einer unerwünschten Spezies unter Schonung der Nicht-Zielorganismen, insbesondere aller Nützlinge.

Auch nach dieser Methode werden nur verhältnismäßig kleine Mengen der Wirkstoffe, welche oft nur Bruchteile der üblichen Dosen der klassischen Insektizidwirkstoffe entsprechen, benötigt (vgl. Birch ed., Pheromones, North Holland Publ. Co., 1974).

Die Methoden 1 und 2 sind insofern nachteilig, als der Lockstoff synthetischer Herkunft seinem natürlichen Vorbild bezüglich Struktur und Reinheit exakt gleichen muß (Minks und Voermann, Entomologia exp. and appl. 16 (1973) 341 bis 49 sowie Wegler, Chemie der Pflanzenschutz- und Schädlingsbekämpfungsmittel (1981) Bd. 6, S. 167). Technische Gemische o.ä. haben bei Fallenfangversuchen regelmäßig versagt.

Aus den nach Methoden 1 und 2 gewonnenen Erfahrungen war anzunehmen, daß auch Methode 3 nur mit hochreinem Pheromon des Apfelwicklers zu verwirklichen sein würde. Daher wurde bisher für das Paarungsstörungsverfahren nur reines 8E,10E-Dodecadienol eingesetzt (G.H.L. Rothschild, Insect. Suppr. Controlled Release Pheromonic Syst. Vol. 2, 117 bis 34).

Es wurde nun gefunden, daß überraschenderweise das Gemisch der 4 Stereoisomeren 8E, 10E-Dodecadienol (Ia), 8E, 10Z-Dodecadienol (Ib), 8Z, 10E-Dodecadienol (Ic) und 8Z, 10Z-Dodecadienol (Id) ebenfalls mit gutem Erfolg für das Paarungsstörungsverfahren eingesetzt werden kann.

Man erhält das verwendete Gemisch besonders vorteilhaft, indem man 1,8-Octandiol (II) in an sich bekannter Weise in einem Lösungsmittel in Gegenwart eines Halogenierungsmittels in ein Halogenderivat der allgemeinen Formel (III) überführt, (III) anschließend mit einem Vinylether der allgemeinen Formel (IV) in Gegenwart einer Säure zum Acetal der allgemeinen Formel (V) umsetzt, (V) in Gegenwart von Magnesium mit Crotonaldehyd in ein Alkenol der allgemeinen Formel (VI) überführt und aus (VI) in Gegenwart von Säure gleichzeitig die Alkoholschutzgruppe abspaltet und die Hydroxyfunktion eliminiert. 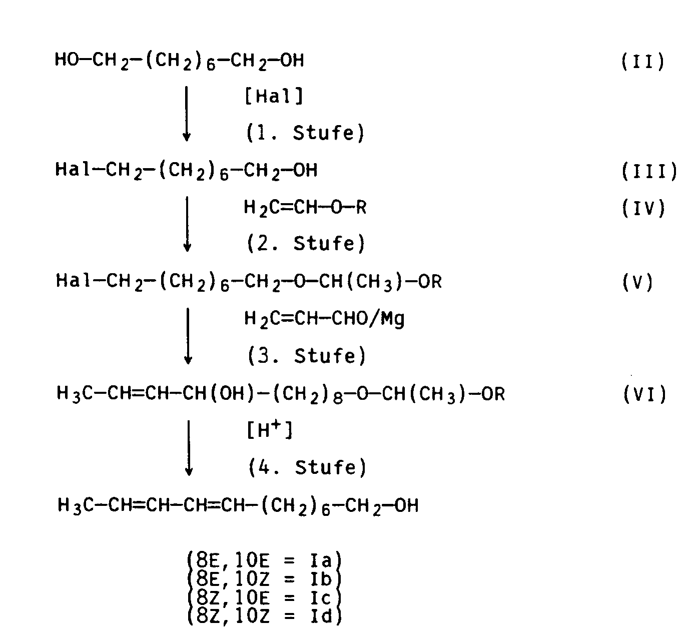
In den Formeln (III) und (V) steht Hal für ein Halogenatom wie Fluor, Chlor, Brom und Iod, vorzugsweise Chlor und Brom. R in den Formeln (IV), (V) und (VI) bedeutet einen C-organischen Rest, vorzugsweise
- Alkyl mit bis zu acht C-Atomen wie insbesondere Methyl, Ethyl, Propyl, 1-Methylethyl, Butyl, 1-Methylpropyl, 2-Methylpropyl und 1,1-Dimethylpropyl,
- Cycloalkyl mit bis zu sechs C-Atomen wie insbesondere Cyclopropyl, Cyclobutyl, Cyclopentyl und Cyclohexyl, und
- Halogenalkyl mit bis zu acht C-Atomen, wie insbesondere in der endständigen Position durch Fluor, Chlor oder Brom ein- oder zweifach substituiertes, gradkettiges Alkyl wie 2-Fluorethyl, 3-Fluorpropyl, 4-Fluorbutyl, 5-Fluorpentyl, 6-Fluorhexyl, 7-Fluorheptyl, 8-Fluoroctyl, 2-Chlorethyl, 3-Chlorpropyl, 4-Chlorbutyl, 5-Chlorpentyl, 6-Chlorhexyl, 7-Chlorheptyl, 8-Chloroctyl, 2-Bromethyl, 3-Brompropyl, 4-Brombutyl, 5-Brompentyl, 6-Bromhexyl, 7-Bromheptyl, 8-Bromoctyl, 2,2-Difluorethyl, 3,3-Difluorpropyl, 4,4-Difluorbutyl, 5,5-Difluorpentyl, 6,6-Difluorhexyl, 7,7-Difluorheptyl, 8,8-Difluoroctyl, 2,2-Dichlorethyl, 3,3-Dichlorpropyl, 4,4-Dichlorbutyl, 5,5-Dichlorpentyl, 6,6-Dichlorhexyl, 7,7-Dichlorheptyl, 8,8-Dichloroctyl, 2,2-Dibromethyl, 3,3-Dibrompropyl, 4,4-Dibrombutyl, 5,5-Dibrompentyl, 6,6-Dibromhexyl, 7,7-Dibromheptyl und 8,8-Dibromoctyl.

Die einzelnen Stufen dieses Herstellungsverfahrens werden wie folgt durchgeführt:

### 1. Stufe

### (Rossi, Synthesis, 1981, 359; Chapman et al., J. Am. Chem. Soc., 100, 4878 (1979))

Diese Umsetzung von (II) mit einem Halogenierungsmittel wird üblicherweise bei Temperaturen von 20°C bis 180°C, vorzugsweise 80°C bis 120°C durchgeführt.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Toluol, o-, m- und p-Xylol sowie Chlorbenzol.

Es können auch Gemische der genannten Lösungsmittel verwendet werden.

Als Halogenierungsmittel kommen Phosphortribromid, Phosphortrichlorid und Thionylchlorid, vorzugsweise Bromwasserstoffsäure und Jodwasserstoffsäure, insbesondere Chlorwasserstoffsäure in Betracht.

Die Halogenierungsmittel werden im allgemeinen in äquimolaren Mengen eingesetzt, sie können aber auch im Überschuß oder gegebenenfalls als Lösungsmittel verwendet werden.

Die Edukte werden im allgemeinen in äquimolaren Mengen miteinander umgesetzt. Bei der Verwendung von Halogenwasserstoffsäuren kann es für die Ausbeute vorteilhaft sein, die Säuren in einem Über- oder Unterschuß bezogen auf Octandiol einzusetzen.

Das für die Herstellung der Derivate (III) benötigte 1,8-Octandiol ist aus der Literatur bekannt (Bouveault et al., Bull. Soc. Chim. Fr. [3] 31, 1204).

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

### 2. Stufe

### (Chládek et al., Chem. Ind., 1964, 171)

Diese Umsetzung von (III) mit (IV) wird üblicherweise bei Temperaturen von -20°C bis 60°C, vorzugsweise 0°C bis 20°C durchgeführt.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Ether wie Tetrahydrofuran und tert.-Butyl-methylether.

Es können auch Gemische der genannten Lösungsmittel verwendet werden. Außerdem kann die Umsetzung auch ohne die Verwendung von Lösungsmitteln durchgeführt werden.

Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren wie Ameisensäure, Essigsäure, Propionsäure, Benzolsulfonsäure, o-, m- und p-Toluolsulfonsäure, Oxalsäure, Zitronensäure und Trifluoressigsäure Verwendung.

Die Säuren werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, den Vinylether in einem Über- oder Unterschuß bezogen auf Chloroctanol einzusetzen.

Die für die Herstellung der Derivate (V) benötigten Vinylether der allgemeinen Formel (IV) sind aus der Literatur bekannt (Reppe, Liebigs Ann. Chem. 601, 84 (1956)) oder können nach den dort beschriebenen Methoden hergestellt werden.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

### 3. Stufe

### (Henze et al., J. Org. Chem. 7, 326 (1942))

Diese Umsetzung von (V) mit Crotonaldehyd in Gegenwart von Magnesium erfolgt üblicherweise bei Temperaturen von -20°C bis 80°C, vorzugsweise 0°C bis 40°C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Ether wie Diethylether und Tetrahydrofuran.

Es können auch Gemische der genannten Lösungsmittel verwendet werden.

Magnesium wird im allgemeinen in äquimolaren Mengen in Form von Spänen eingesetzt, es kann aber auch im Überschuß von 0,1 bis 100 mol-%, vorzugsweise 0,1 bis 50 mol-%, insbesondere 0,1 bis 20 mol-%, verwendet werden.

Die Edukte (V und Crotonaldehyd) werden im allgemeinen in äquimolaren Mengen miteinander umgesetzt. Es kann für die Ausbeute vorteilhaft sein, Crotonaldehyd in einem Überschuß von 0,1 bis 50 mol-%, vorzugsweise 0,1 bis 20 mol-%, insbesondere 0,1 bis 10 mol-%, bezogen auf (V) einzusetzen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

### 4. Stufe

### (Chavamie et al., Bull. Soc. Chim. Belg., 22, 410 (1908))

Die Abspaltung der Alkoholschutzgruppe und die gleichzeitige Eliminierung der Hydroxyfunktion wird üblicherweise bei Temperaturen von -20°C bis 150°C, vorzugsweise 40°C bis 80°C durchgeführt.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, sowie hochsiedende Verbindungen wie Phthalsäuredi-C₁-C₈-alkylether wie Phthalsäuredimethylester, Phthalsäurediethylester, Phthalsäuredipropylester, Phthalsäuredi-1-methylethylester, Phthalsäuredibutylester, Phthalsäuredipentylester, Phthalsäuredihexylester, Phthalsäuredioctylester und Phthalsäuredi-2-ethylhexylester, besonders bevorzugt Phthalsäuredibutylester, Phthalsäuredipentylester, Phthalsäuredihexylester und Phthalsäuredi-2-ethylhexylester.

Es können auch Gemische der genannten Lösungsmittel verwendet werden.

Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren wie Ameisensäure, Essigsäure, Propionsäure, Oxalsäure, Zitronensäure und Trifluoressigsäure Verwendung.

Die Säuren werden im allgemeinen in katalytischen Mengen eingesetzt, sie können aber auch äquimolar, im Überschuß oder gegebenenfalls als Lösungsmittel verwendet werden.

Als besonders vorteilhaft für die Synthese der Dodecadienole (Ia) bis (Id) aus VI hat sich die Umsetzung von VI mit Acetanhydrid und Schwefelsäure [Chavamie, van Roelen, Bull. Soc. Chim. Belg. 22, 410 (1908)] in cyclischen und acyclischen Ethern, wie Diethylether, Tetrahydrofuran oder Dioxan, aromatischen Kohlenwasserstoffen wie Benzol, Toluol oder Xylol, aliphatischen Kohlenwasserstoffen wie Pentan, Hexan, Heptan oder Cyclohexan bzw. halogenierten Kohlenwasserstoffen wie Dichlormethan, Dichlorethan, Trichlorethan bei Temperaturen zwischen -20°C und 100°C erwiesen.

Dabei wird in einem Reaktionsschritt Wasser eliminiert und die Schutzgruppe abgespalten.

Das auf diese Weise erhaltene Gemisch kann man einer Isomerisierungsreaktion unterwerfen, um den Anteil an 8E,10E-Dodecadienol zu erhöhen.

Hierzu erhitzt man mit katalytischen Mengen 4-Chlorthiophenol, 3-Chlorthiophenol, Thiophenol oder Mercaptoessigsäure und wäscht anschließend den Katalysator durch Behandeln mit Natronlauge aus.

Die Isomerisierung läßt sich ebenfalls durch UV-Bestrahlung in Gegenwart von Jod oder Diphenyldisulfid bewerkstelligen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen-und Endprodukte als Feststoffe erhalten werden, kann die Reinigung auch durch Umkristallisieren oder Digerieren erfolgen.

Das nach diesem Verfahren gewonnene Gemisch der Verbindungen (Ia), (Ib), (Ic) und (Id) eignet sich zur Bekämpfung des Apfelwicklers Cydia pomonella nach der Paarungsstörungsmethode.

Das Gemisch kann zusammen mit üblichen Hilfsstoffen, z.B. entsprechend präparierten Streifen aus Kunststoff, Bindegarnen, lockstoffgefüllten Ampullen o.ä. (beispielsweise gemäß DE-A 41 01 878) angewendet werden und auch herstellungsbedingte Verunreinigungen enthalten.

Zur Formulierung des Gemisches kommen sowohl flüssige wie auch feste Präparationen in Frage. Als Lösungsmittel kommen hochsiedende, aromatische, aliphatische oder cycloaliphatische Verbindungen in Betracht. Neben Kohlenwasserstoffen eignen sich Ester, Ether oder Ketone besonders gut. Typische Vertreter dieser Klassen sind z.B. Xylol, Methylnaphthaline, Paraffinöle, Cyclohexanon, Ethylglykolacetat, Isophoron und Dibutylphthalat. Diese Lösungsmittel können allein oder in Mischungen mit anderen Komponenten Verwendung finden. Die den Verbindungen Ia bis Id entsprechenden gesättigten C₁₂-Alkohole und C₁₂-Ester sowie deren Homologe sind besonders geeignete Formulierungshilfsmittel und können als Synergisten angesehen werden, da sie die Wirkung von Ia, Ic und Td verstärken.

Weiterhin können Lösungen in pflanzlichen, tierischen oder synthetischen Ölen oder Fetten und anderen verdunstungshemmenden Lösungsmitteln mit niedrigem Dampfdruck wie z.B. Dioctylphthalat zum Zwecke der Wirkungsverlängerung hergestellt werden.

Des weiteren ist es möglich, das Gemisch in oder an natürliche oder synthetische feste Träger wie Gummi, Kork, Zellulose, Kunststoffe, gemahlene Kohle, Holzmehl, Silikate, Bimskies, gebrannten Ton oder ähnliche feste Trägerstoffe zu binden oder in speziellen Kapselformulierungen oder Kunststoffbehältern einzusetzen, um so eine gleichmäßige Abgabe an die Luft über längere Zeiträume hinweg zu erreichen. Außerdem kann der Wirkstoff aus geeigneten Behältern, z.B. Kapillaren, Schläuchen (Gummi) oder anderen Gefäßen, durch enge Öffnungen oder durch Diffusion durch die Behälterwand sowie aus mehrschichtigen Kunststoffplättchen, sogenannten Flakes, zur Verdunstung gebracht werden, wodurch über längere Zeiträume hinweg besonders gleichmäßige Duftkonzentrationen erzielt werden.

Der Gehalt dieser Zubereitungen an Gemisch kann innerhalb weiter Grenzen schwanken. Generell kann das Verhältnis Wirkstoff : Zusatzstoff z.B. im Bereich von 10 : 1 bis 1 : 10³ liegen. In Kapselformulierungen oder anderen geeigneten Behältern kann z.B. der Wirkstoff in reiner, unverdünnter Form angewendet werden und sein Gewichtsanteil, bezogen auf die Gesamtformulierung, sehr hoch sein und bis zu 90 % betragen. Im allgemeinen genügen jedoch sehr geringe Wirkstoffkonzentrationen in den Zubereitungen, um die gewünschte Wirkung auf Apfelwickler-Männchen auszuüben. Bevorzugt ist ein Mengenverhältnis
Wirkstoff : Zusatzstoff von 1 : 3 bis 1 : 10² insbesondere 1 : 10 bis 1 : 100.

### Herstellungsbeispiele

### Beispiel 1

250 ml tert.-Butyl-methylether wurden getrennt aber gleichzeitig mit einer Mischung aus 40 g 8-Chloroctanol, 0,1 g Essigsäure und 60 g tert.-Butyl-methylether und einer Mischung aus 17 g Ethylvinylether und 83 g tert.-Butyl-methylether versetzt. Nach beendeter Zugabe wurde die Reaktionsmischung 12 h bei 20°C gerührt. Zur Aufarbeitung wurde die Reaktionsmischung dreimal mit je 300 ml gesättigter Natriumhydrogencarbonatlösung gewaschen, die organische Phase wurde mit Natriumsulfat getrocknet und eingeengt. Man erhielt das Chloracetal in einer Ausbeute von 235 g (85%ig) = 84 %.

### Beispiel 2

260 g 8-Chloroctylethoxy-1-ethylether und 35 g Magnesium werden in 2 l THF in üblicher Weise mit 77 g Crotonaldehyd bei -10°C langsam versetzt. Man läßt 1 Std. bei -10°C nachrühren, hydrolysiert mit 2 l gesättigter Ammoniumchloridlösung und trennt die organische Phase ab. Die Wasserphase wird mit Toluol mehrfach extrahiert und das Extrakt zusammen mit der Hauptmenge über Natriumsulfat getrocknet und eingeengt. Das Rohprodukt wird durch eine Molekulardestillation bei einer Temperatur von 190°C und 1 mbar gereinigt.

Ausbeute: 259 g (85%ig) = 80 %

### Beispiel 3

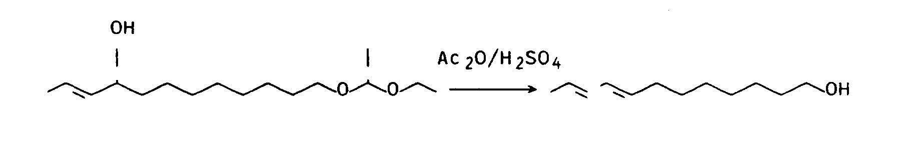

Zu einer auf 0°C gekühlten Lösung von 33 g Acetanhydrid und 122 g 9-Hydroxydodec-10-enylethoxy-1-ethylether in 300 ml Dioxan gibt man 3,15 g konzentrierte Schwefelsäure und läßt 1,5 Std. bei 0°C rühren. Anschließend gibt man eine Lösung bestehend aus 225 ml einer 13,25%igen Natriumchloridlösung und 4 g Natriumhydroxid zu und rührt 20 Min. bei 0 bis 10°C. Die organische Phase wird isoliert, getrocknet und mit 1 g 4-Chlorthiophenol 4 Std. bei 100°C gerührt. Man extrahiert einmal mit 25 ml 25%iger Natronlauge, wäscht zweimal mit Wasser neutral und destilliert die organische Phase bei vermindertem Druck.

Ausbeute: 55 g ≙ 80 % (Isomerengemisch, Anteil des E,E-Isomeren: 45 %) Sdp.: 120°C/0,1 mbar.

Wie in Beispiel 1 beschrieben können die folgenden Halogenacetale V hergestellt werden: 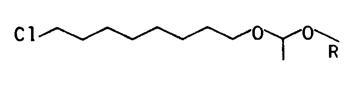
R = Methyl, Ethyl, Propyl, Isopropyl, Butyl, Isobutyl, Pentyl, Hexyl, Cyclohexyl, 8-Halogenoctyl.

Wie in Beispiel 2 beschrieben können die folgenden Alkenole VI hergstellt werden: 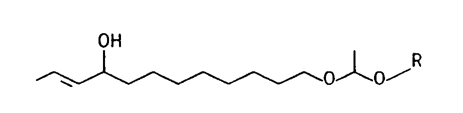
R = Methyl, Ethyl, Propyl, Isopropyl, Butyl, Isobutyl, Pentyl, Hexyl, Cyclohexyl, 9-Hydroxydodec-10-enyl.

### Anwendungsbeispiele

Die Wirksamkeit neuer Pheromonprodukte bei der Methode der Paarungsstörung läßt sich nur in großflächigen Versuchen bestimmen, da aussagefähige Labortests zur Zeit noch nicht zur Verfügung stehen.

Eingesetzt wurde ein Isomerengemisch, das wie in Beispiel 3 beschrieben hergestellt wurde und die folgende Zusammensetzung hat:
8E, 10E-Dodecadienol 45 %
Summe der anderen Isomere 40 %
Der Wirkstoff wurde in Polyethylenampullen abgefüllt. Eine Ampulle enthält dabei ca. 500 mg des erfindungsgemäßen Produktes, die zweite ca. 400 mg 11Z-Tetradecenylacetat, Pheromon des Fruchtschalenwicklers Adoxophyes orana. Die Ampullen werden vor Flugbeginn der Schädlinge gleichmäßig in den Apfelanlagen angebracht (ca. 500 Ampullen/ha) (Tabelle 1).

**Tabelle 1**

| Standort | Fläche ha | Befall Apfelwickler | Befall Fruchtschalenwickler |
|---|---|---|---|
| Bölingen | 3,5 | + | + |
| Krefeld | 3,0 | + | + |
| Bauschlott | 6,0 | - | - |
| Dieblich | 1,0 | + | + |
| Bad Hönig | 4,5 | + | + |

Dabei wurde nur in einer Fläche das vorgegebene Ziel nicht erreicht.

An 8 Standorten wurde als Vergleich das bekannte 8E, 10E-Dodecadienol ausgebracht, ebenso 500 mg/Ampulle und in Kombination mit dem Fruchtschalenwicklerpheromon. An drei Standorten wurde im Verlauf der Saison die wirtschaftliche Schadschwelle von 1 % Befall überschritten (Tabelle 2).

**Tabelle 2**

| Standort | Fläche ha | Befall Apfelwickler | Befall Fruchtschalenwickler |
|---|---|---|---|
| Kriftel | 2,0 | + | + |
| Mainz | 2,0 | + | + |
| Dieblich | 3,0 | + | - |
| Urmitz | 0,4 | + | + |
| Bauschlott | 6,0 | - | - |
| Geisenheim | 3,0 | - | - |
| Bölingen | 3,4 | - | - |
| Krefeld | 3,0 | + | + |
| + Befall unter Schadschwelle (1 %) - Befall über Schadschwelle (1 %) | | | |

Es konnte somit gezeigt werden, daß in praxisrelevanten großflächigen Versuchen das Isomerengemisch einen ausreichenden Wirkungsgrad erzielt. Mit dem bekannten Wirkstoff wurden überraschenderweise eher schlechtere Ergebnisse erhalten, als mit dem erfindungsgemäßen Isomerengemisch.

Ein weiterer Vorteil des neuen Verfahrens besteht darin, daß das Isomerengemisch der 8, 10-Dodecadienole ohne Formulierungshilfen verwendet werden kann. Bisher mußte der reine Wirkstoff 8E,10E-Dodecadienol zusammen mit Formulierungshilfsmittel wie z.B. gesättigte C₁₂-Ester und Homologe eingesetzt werden, um konstante Abdampfraten zu erzielen. Die Möglichkeit zum Verzicht auf Formulierungshilfen bedeutet einen zusätzlichen Vorteil.

## Patentansprüche

1. Verfahren zur Herstellung eines Gemisches von 8E,10E-Dodecadienol (Ia), 8E,10Z-Dodecadienol (Ib), 8Z,10E-Dodecadienol (Ic) und 8Z,10Z-Dodecadienol (Id),
H₃C-CH=CH-CH=CH-(CH₂)₆-CH₂-OH
(8E,10E = Ia)
(8E,10Z = Ib)
(8Z,10E = Ic)
(8Z,10Z = Id)
dadurch gekennzeichnet, daß man 1,8-Octandiol (II)
HO-CH₂-(CH₂)₆-CH₂-OH (II)
in an sich bekannter Weise in einem Lösungsmittel in Gegenwart eines Halogenierungsmittels in ein Halogenderivat der allgemeinen Formel (III),
Hal-CH₂-(CH₂)₆-CH₂-OH (III)
in der Hal für ein Halogenatom steht, überführt, (III) anschließend mit einem Vinylether der allgemeinen Formel (IV),
H₂C=CH-O-R (IV)
in der R für einen C-organischen Rest steht, in an sich bekannter Weise in Gegenwart einer Säure zu einem Acetal der allgemeinen Formel (V)
Hal-CH₂-(CH₂)₆-CH₂-O-CH(CH₃)-OR (V)
umsetzt, (V) in Gegenwart von Magnesium mit Crotonaldehyd in ein Alkenol der allgemeinen Formel (VI)
H₃C-CH=CH-CH(OH)-(CH₂)₈-O-CH(CH₃)-OR (VI)
überführt und aus (VI) in Gegenwart von Säure gleichzeitig die Alkoholschutzgruppe abspaltet und die Hydroxyfunktion eliminiert.

2. Alkenole der allgemeinen Formel (VIa),
H₃C-CH=CH-CH(OH)-(CH₂)₈-O-CH(CH₃)-OR² (VIa)
in der R² für C₁-C₈-Alkyl, C₃-C₆-Cycloalkyl, C₁-C₈-Halogenalkyl und 9-Hydroxydodec-10-enyl steht.

3. Verfahren zur Bekämpfung des Apfelwicklers Cydia pomonella nach der Paarungsstörungsmethode, dadurch gekennzeichnet, daß man ein Mittel enthaltend ein Gemisch aus 8E,10E-Dodecadienol (Ia), 8E,10Z-Dodecadienol (Ib), 8Z,10E-Dodecadienol (Ic) und 8Z,10Z-Dodecadienol (Id) in einer solchen Menge anwendet, daß die männlichen Tiere der Art bei der Auffindung der weiblichen Tiere gestört werden.

4. Verfahren nach Anspruch 3, dadurch gekennzeichnet, daß das angewendete Mittel zu mindestens 40 % aus einem Gemisch von (Ia), (Ib), (Ic) und (Id) besteht.

## Claims

1. A process for preparing a mixture of 8E,10E-dodecadienol (1a), 8E,10Z-dodecadienol (Ib), 8Z,10E-dodecadienol (Ic) and 8Z,10Z-dodecadienol (Id),
H₃C-CH=CH-CH=CH-(CH₂)₆-CH₂-OH
(8E,10E = Ia)
(8E,10Z = Ib)
(8Z,10E = Ic)
(8Z,10Z = Id)
which comprises converting 1,8-octanediol (II)
HO-CH₂-(CH₂)₆-CH₂-OH (II)
in a conventional manner in a solvent in the presence of a halogenating agent into a halo derivative of the formula (III)
Hal-CH₂-(CH₂)₆-CH₂-OH (III)
where Hal is halogen, subsequently reacting (III) with a vinyl ether of the formula (IV)
H₂C=CH-O-R (IV)
where R is a C-organic radical, in a conventional manner in the presence of an acid to give an acetal of the formula (V)
Hal-CH₂-(CH₂)₆-CH₂-O-CH(CH₃)-OR (V)
,
converting (V) in the presence of magnesium and crotonaldehyde into an alkenol of the formula (VI)
H₃C-CH=CH-CH(OH)-(CH₂)₈-O-CH(CH₃)-OR (VI)
,
and simultaneously eliminating from (VI) in the presence of acid the alcohol protective group and the hydroxyl group.

2. An alkenol of the formula (VIa)
H₃C-CH=CH-CH(OH)-(CH₂)₈-O-CH(CH₃)-OR² (VIa)
where R² is C₁-C₈-alkyl, C₃-C₆-cycloalkyl or C₁-C₈-haloalkyl or 9-hydroxy-10-dodecenyl.

3. A method for controlling the codling moth Cydia pomonella by interfering with mating, which comprises applying a composition containing a mixture of 8E,10E-dodecadienol (Ia), 8E,10Z-dodecadienol (Ib), 8Z,10E-dodecadienol (Ic) and 8Z,10Z-dodecadienol (Id) in an amount sufficient to interfere with the finding of the females by the males of the species.

4. A method as claimed in claim 3, wherein the applied composition is at least 40% composed of a mixture of (Ia), (Ib), (Ic) and (Id).

## Revendications

1. Procédé de préparation d'un mélange de 8E, 10E-dodécadiénol (Ia), 8E, 10Z-dodécadiénol (Ib), 8Z, 10E-dodécadiénol (Ic) et 8Z, 10Z-dodécadiénol (Id),
H₃C-CH=CH-CH=CH-(CH₂)₆-CH₂-OH
(8E,10E = Ia)
(8E,10Z = Ib)
(8Z,10E = Ic)
(8Z,10Z = Id)
caractérisé en ce que l'on convertit le 1,8-octane-diol (II)
HO-CH₂-(CH₂)₆-CH₂-OH (II)
de manière connue en soi, dans un solvant, en présence d'un agent halogénant, en un dérivé halogéné de formule générale III
Hal-CH₂-(CH₂)₆-CH₂-OH (III)
dans laquelle Hal représente un atome d'halogène, qu'on fait réagir ensuite avec un éther vinylique de formule générale IV
H₂C=CH-O-R (IV)
dans laquelle R représente un radical organique carboné, de manière connue en soi, en présence d'un acide, ce qui donne un acétal de formule générale V
Hal-CH₂-(CH₂)₆-CH₂-O-CH(CH₃)-OR (V)
qu'on convertit à l'aide de l'aldéhyde crotonique en présence de magnésium en un alcénol de formule générale VI
H₃C-CH=CH-CH(OH)-(CH₂)₈-O-CH(CH₃)-OR (VI)
d'où l'on élimine simultanément, en présence d'un acide, le groupe protecteur de la fonction alcool et la fonction hydroxy.

2. Alcénols de formule générale VIa
H₃C-CH=CH-CH(OH)-(CH₂)₈-O-CH(CH₃)-OR² (VIa)
dans laquelle R² représente un groupe alkyle en C1-C8, cycloalkyle en C3-C6, halogéno-alkyle en C1-C8 ou 9-hydroxydodéca-10-ényle.

3. Procédé pour combattre le parasite de la pomme Cydia pomonella par perturbation des accouplements, caractérisé en ce qu'on l'on utilise un produit contenant un mélange de 8E, 10E-dodécadiénol (Ia), 8E, 10Z-dodécadiénol (Ib), 8Z, 10E-dodécadiénol (Ic) et 8Z, 10Z-dodécadiénol (Id) en quantité efficace pour perturber la recherche des femelles par les mâles de l'espèce.

4. Procédé selon la revendication 3, caractérisé en ce que le produit utilisé consiste pour au moins 40% en un mélange de (Ia), (Ib), (Ic) et (Id).
